# EUROPÄISCHE PATENTSCHRIFT

(11) **EP 3 620 195 B1**
(45) Veröffentlichungstag und Bekanntmachung des Hinweises auf die Patenterteilung: **13.04.2022**
(21) Anmeldenummer: 19189096.1
(22) Anmeldetag: 30.07.2019
(51) Int. Cl.: A61M 16/04, A61M 16/00, A61M 39/24, F16K 15/14

(54) **BEATMUNGSVORRICHTUNG**
VENTILATION DEVICE
DISPOSITIF DE RESPIRATION

(30) Priorität: 23.08.2018 DE 102018120533
(43) Veröffentlichungstag der Anmeldung: 11.03.2020
(73) Patentinhaber: CEGLA Medizintechnik GmbH & Co. KG, 56410 Montabaur (DE)
(72) Erfinder: Ebinger, Andrea, 56410 Montabaur (DE); Cegla, Ulrich, 56410 Montabaur (DE)
(74) Vertreter: Engelhardt, Volker

(56) Entgegenhaltungen:
- EP-A1- 2 489 413
- WO-A1-2018/072036
- WO-A2-98/07401
- US-A1- 2017 361 056

## Beschreibung

Die Erfindung bezieht sich auf eine Beatmungs-Vorrichtung nach dem Oberbegriff des Patentanspruchs 1.

Solche Beatmungs-Vorrichtungen sind seit langen Jahren bekannt und werden üblicherweise bei Lebewesen, insbesondere Menschen, eingesetzt, die nicht mehr selbstständig atmen können. Durch das der Beatmungs-Vorrichtung zugeordnete Beatmungsgerät wird ein intermittierender Luftstrom erzeugt, der beispielsweise auch mit Sauerstoff und anderen Gasen angereichert sein kann. Dieser Luftstrom ist über einen Beatmungsschlauch und einem Luftverteiler in einen an diesen angeschlossenen Tubus weitergeleitet, der in dem Lungenbereich des Patienten eingeführt ist. Somit gelangt die von dem Beatmungsgerät erzeugte Atemluft in die Lunge des Patienten. Beim Ausatmen des Patienten wird die verbrauchte Atemluft durch den Tubus in den Luftverteiler und von diesem zurück in das Beatmungsgerät gesaugt.

Es hat sich bei solchen Behandlungssituationen herausgestellt, dass der Lungenbereich des Patienten verschleimt, und zwar insbesondere dann, wenn der Patient über einen längeren Zeitraum zu beatmen ist. Durch die Verschleierung der Lunge können jedoch Infektionen und Atembeschwerden entstehen, die lebensbedrohend sein können.

Aus der EP 2 489 413 B1 ist zwar ein Therapiegerät zur Behandlung von Atemwegserkrankungen bei Patienten bekannt geworden, jedoch können die bislang bekannten Atemwegstherapiegeräte ausschließlich für Patienten mit selbstständiger Atmung verwendet werden. Für Patienten, die nicht mehr selbstständig atmen können und daher an ein bekanntes Beatmungsgerät anzuschließen sind, können solche Therapiegeräte nicht verwendet werden. Durch die bei der Ein- und/oder Ausatmung erzeugten Luftwiderstände innerhalb des Therapiegerätes entstehen oszillierende Luftdruckschwankungen, die in den Bronchial- und Lungenbereich des Patienten übertragen sind und dort schleimlösend wirken. Folglich kann der gelöste Schleim aus der Lunge selbstständig ausgeartet bzw. abtransportiert werden. Die sogenannten PEP oder OPeP-Therapiegeräte, durch die positive expiratory pressure (PEP) oder oscillating positive expiratory pressure(OPEP) Beatmungsbehandlungen durchführbar ist, erzeugen nämlich einen positiven und negativen Beatmungsdruck, durch den der sich in der Lunge abgelagerter Schleim lösen und aus dieser abtransportieren lässt.

Aus der EP 2 489 413 A1 ist eine Beatmungsvorrichtung bestehend aus einem Beatmungsgerät mit einem Luftausgangs- und Lufteingangskanal bekannt geworden. In diesen beiden Kanälen sind jeweils ein Schlauch eingesetzt, der mit einer der beiden Kanäle verbunden ist. Durch einen Luftverteiler, an dem die Öffnungen vorgesehen sind, die mit einer der beiden Schläuche des Beatmungsgerätes und mit einem in den Mund- oder Nasenraum des Lebewesens einsetzbaren Tubus zusammenwirken, kann beim Ein- bzw. Ausatmen die Luftströmung automatisiert gesteuert sein.

Durch die Vibrationen der beiden Schläuche während des Einatmungs- bzw. Ausatmungsvorganges werden die in der Lunge oder den Rachenräumen eingelagerten Schleimpartikel gelöst und aus diesen abtransportiert.

Nachteiligerweise sind solche Beatmungsvorrichtungen für Lebewesen, die künstlich zu beatmen sind, da diese nicht mehr selbstständig bzw. eigenständig die Atmung ausführen können, nicht geeignet, denn die selbstständige Atmung ist zwingend erforderlich, um das dort beschriebene Beatmungsgerät überhaupt benutzen zu können.

Die Verbindung der bekannten Atemwegstherapiegeräte, die nach dem vorstehend erläuterten Behandlungsprinzip arbeiten und den bekannten Beatmungs-Vorrichtungen sind nicht bekannt. Vielmehr wird der in den Lungen der nicht selbstständig atmenden Patienten operativ oder durch andere medizinische Maßnahmen gelöst und abtransportiert, durch die jedoch eine hohe Infektionsgefahr oder Atemstillstand besteht, da der Patient - zumindest kurzfristig - von dem Beatmungsgerät abzukoppeln ist und fremde Gegenstände für die Schleimlösung und dessen Abtransport einzusetzen sind.

Dokument US 2017/361056 offenbart auch ein Atemwegstherapiegerät für selbständig atmende Patienten um ihre Atmungsorgane sowohl bei der Inspiration als auch bei der Exspiration durch oszillierende Schwingungen des Luftstroms zu trainieren.

Es ist daher Aufgabe der Erfindung die eingangsgenannten Beatmungs-Vorrichtungen derart weiterzubilden, dass ein zu beatmender Patient mit kostengünstigen Maßnahmen eine Schleimlösung und dessen Abtransport aus dem Lungenbereich erfährt, ohne dass hierfür Fremdgegenstände oder ein Abschalten der Beatmungs-Vorrichtung erforderlich ist.

Diese Aufgabe ist erfindungsgemäß durch die Merkmale des kennzeichnenden Teils von Patentanspruch 1 gelöst.

Weitere vorteilhafte Weiterbildungen der Erfindung ergeben sich aus den Unteransprüchen.

Dadurch, dass in dem Einatmungsschlauch ein Atemwegstherapiegerät eingesetzt ist, dass durch das Beatmungsgerät ein Luftstrom generiert ist, durch den in dem Atemwegstherapiegerät eine intermittierende Luftdruck-Schwankung entsteht, und dass die derart erzeugten Luftdruck-Schwankungen in dem Bronchial- und Lungenbereich der Lebewesens weitergeleitet oder übertragen sind, ist erreicht, dass sowohl positive als auch negative Luftdrücke auf die Lungenbläschen einwirken, wodurch Schleimablagerungen gelöst und abtransportiert sind. Folglich kann die erfindungsgemäße Beatmungs-Vorrichtung auch für Lebewesen verwendet sein, die keine selbstständige Atmung besitzen, sondern vielmehr medizinische Hilfe in Form einer künstlichen oder maschinellen Beatmung benötigen. Eine solche medizinische Behandlungsmaßnahme führt vorteilhafter Weise dazu, dass die an der Beatmungs-Vorrichtung angeschlossenen Patienten keine oder lediglich eine sehr geringe Schleimablagerung in ihrem Bronchial- und Lungenbereichen aufweisen, da die permanenten Luftdruckschwankungen, die von dem von dem Beatmungsgerät erzeugte Luftstrom und der konstruktiven Ausgestaltung des Atemwegstherapiegerät entstehen, die Auflösung des Schleimes bewirken und gleichzeitig diese Schleimpartikel von dem Patienten aus geatmet bzw. ausgeatmet werden können.

Dabei kann das Atemwegstherapiegerät sowohl aus einem gekrümmten Rohr, in dem ein frei beweglicher Schlauch befestigt ist, als auch als Ventil in Form einer Drosselklappe oder einer Kugel ausgestaltet sein. Entscheidend für die Erzeugung von positiven und negativen Luftdruckschwankungen innerhalb des Atemwegstherapiegerät soll dabei sein, dass die gleichmäßig von dem Beatmungsgerät erzeugte und in das Rohr des Atemwegstherapiegerätes eingepresste Atemluft oszillierend in Ihrem Druckniveau schwankt, wodurch die entsprechenden Luftdruckschwankungen innerhalb der Lunge oder Bronchien des Patienten entstehen. Diese derart erzeugten oszillierenden Luftbrücke führen nämlich dazu, dass sich Fremdpartikel, insbesondere Schleim, von den Lungenbläschen lösen und mit der ausgesaugten Atemluft nach außen abtransportiert sind.

Da ein Patient oftmals die oszillierenden von dem Atemwegstherapiegerät erzeugten Luftdruckschwankungen nicht permanent benötigt, ist es vorteilhaft, wenn das Atemwegstherapiegerät aus dem Beatmungsschlauch der Beatmungs-Vorrichtung ohne Zeitverlust und unkompliziert aus- bzw. eingebaut werden kann. Zu diesem Zweck sind die beiden Enden des Rohrstückes mit entsprechenden Befestigungsadaptern ausgestattet, die in dazu korrespondierende Befestigungsadapter, die dem Beatmungsschlauch zugeordnet bzw. an diesem angebracht sind, zusammensteckbar, so dass das Rohrstück mit den freien Enden des Beatmungsschlauch zusammen geknipst oder mittels eines Gewindes verbunden werden kann.

Vorteilhafterweise sind dabei die Befestigungsadapter an dem Beatmungsschlauch derart ausgestaltet, dass diese luftdicht zusammengesetzt werden können, wenn das Atemwegstherapiegerät aus dem Beatmungsschlauch entfernt ist.

Eine besonders vorteilhafte Weiterbildung der Erfindung besteht darin, parallel zu dem Atemwegstherapiegerät eine Bypass-Leitung vorzusehen, die über ein Ventil ansteuerbar ist. Wenn nämlich das Ventil in einer geöffneten Stellung positioniert ist, strömt die von dem Beatmungsgerät erzeugte Luft unmittelbar durch die Bypass-Leitung zu dem jeweiligen Patienten. Ist das Ventil dagegen geschlossen, so strömt die von dem Beatmungsgerät erzeugte Luft durch das Atemwegstherapiegerät, wodurch die gewünschten intermittierenden Luftdruckschwankungen erzeugt sind, die in die Bronchien bzw. den Lungenbereichen des Lebewesens gelangen und dort die Schleimlösung bewirken.

In der Zeichnung sind zwei erfindungsgemäße Ausführungsbeispiele einer Beatmungs-Vorrichtung dargestellt, die nachfolgend näher erläutert ist. Im Einzelnen zeigt:
- Figur 1: eine erste Ausführungsvariante einer Beatmungs-Vorrichtung bestehend aus einem Beatmungsgerät und zwei an diesem befestigten Beatmungsschläuche in der ein Atemwegstherapiegerät eingesetzt ist, aus einem Luftverteiler an dem die Beatmungsschläuche angeschlossen sind sowie einem dem Luftverteiler zugeordneten Tubus, der in den Lungenbereich eines Lebewesens eingeführt ist, in Seitenansicht,
- Figur 2: einen vergrößerten Ausschnitt der Beatmungs-Vorrichtung gemäß Figur 1 mit einem in den Beatmungsschlauch eingesetzten Atemwegsthera-piegerät, im Schnitt,
- Figur 3a: den befestigten Zustand des Atemwegstherapiegerät an dem Beat-mungsschlauch gemäß Figur 2,
- Figur 3b: den gelösten Zustand zwischen dem Beatmungsschlauch und dem Atemwegstherapiegerät gemäß Figur 3a,
- Figur 4a: eine zweite Ausführungsvariante einer Beatmungs-Vorrichtung bestehend aus einem Beatmungsgerät und einem Beatmungs- und einem Ausatmungsschlauch, welcher den Ausatmungsschlauch umschlingt, und aus einem in den Beatmungsschlauch eingesetzten Atemwegsthe-rapiegerät, im Schnitt,
- Figur 4b: die Verbindung zwischen dem Beatmungsschlauch und dem Atem-wegstherapiegerät gem. Figur 4 entlang der Schnittlinie IVb - IVb und
- Figur 5: die Vorrichtung gemäß Figur 1 mit einer Bypass-Leitung und einem Ventil, durch die das Atemwegstherapiegerät an das Beatmungsgerät anschließbar oder getrennt ist.

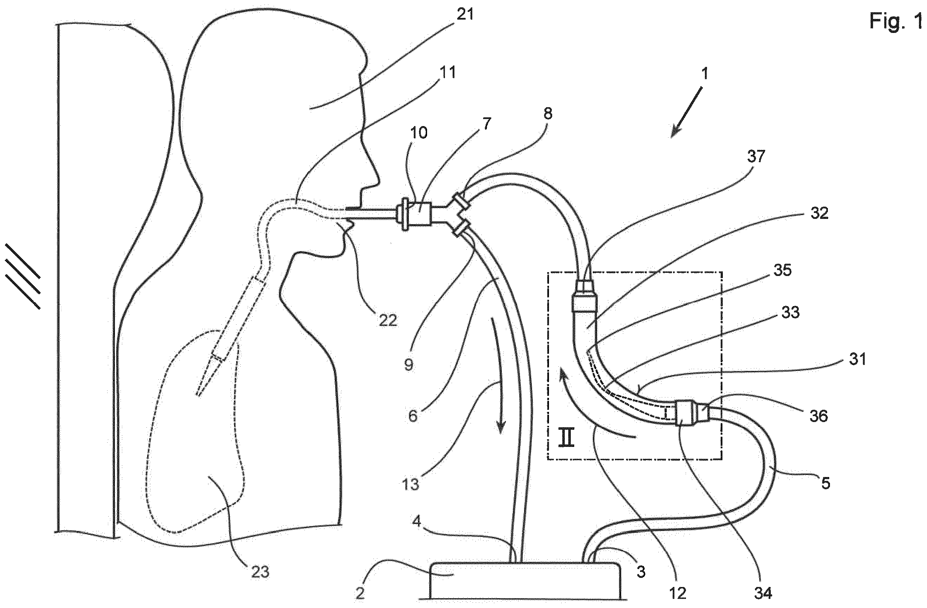

Aus Figur 1 ist eine Beatmungs-Vorrichtung 1 zu entnehmen, durch die ein Lebewesen 21, beispielsweise ein Mensch oder ein Tier, beatmet werden soll, da das Lebewesen 21 selbstständig nicht mehr atmen kann.

Die Beatmungs-Vorrichtung 1 besteht dabei aus einem Beatmungsgerät 2, durch das Atemluft generiert und in Richtung eines Luftausgangs-Kanal 3 geleitet ist. Darüber hinaus ist an dem Beatmungsgerät 2 ein Lufteingangs-Kanal 4 vorgesehen. An dem Luftausgangs-Kanal 3 ist ein Beatmungs-Schlauch 5 und an dem Lufteingangs-Kanal 4 ist ein Ausatmungs-Schlauch 6 befestigt.

Der Beatmungsschlauch 5 kann auch als Einatemschenkel oder als Coaxialschlauch bezeichnet sein.

Darüber hinaus ist der Beatmungs-Vorrichtung 1 ein Luftverteiler 7 zugeordnet, der drei Öffnungen 8, 9 und 10 aufweist. An der Öffnung 8 ist der Beatmungs-Schlauch 5, an der Öffnung 9 der Ausatmungs-Schlauchs 6 und an der Öffnung 10 ein Tubus 11 angeschlossen. Der Tubus 11 ist dabei in den Lungenbereich 23 durch den Mundraum 22 des Lebewesens 21 eingeführt, so dass die von dem Beatmungsgerät 2 erzeugte Atemluft 18 durch den Beatmungsschlauch 5, den Luftverteiler 7 und den Tubus 11 in die Lunge 23 eingepresst ist, wodurch der Patient 21 künstlich beatmet wird. Die verbrauchte Atemluft 13 wird innerhalb eines vorgegebenen Zyklus durch das Beatmungsgerät 2 aus der Lunge 23 herausgesaugt und über den Tubus 11, den Luftverteiler 7 gelang diese in den Ausatmungs-Schlauch 6.

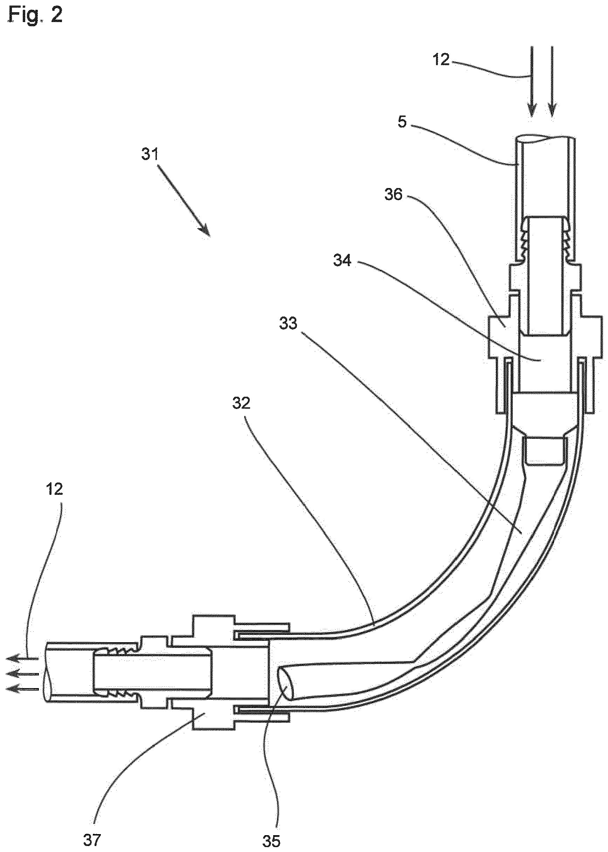

In Figur 2 ist ein in dem Beatmungs-Schlauch 5 eingebautes Atemwegstherapiegerät 31 zu entnehmen. Das Atemwegstherapiegerät 31 besteht aus einem gekrümmten Rohr 32, in dem ein Schlauch 33 vorgesehen ist. Dabei ist der Schlauch 33 mit einem Ende an der Atemluft-Eingangsseite 34 des Atemwegstherapiegerätes lageorientiert an dem Rohr 22 gehalten, und zwar derart, dass die in das Rohr 32 eingepresst Atemluft 12 in das Innere des Schlauches 33 gelangt. Das gegenüberliegende freie Ende des Schlauches 33 kann folglich innerhalb des Rohres 32 frei schwingen. Durch die Krümmung des Rohres 32 und die Bewegungsmöglichkeiten des Schlauches 33 entsteht eine oszillierenden Luftdruckschwankung, die von dem Rohr 32 in die Fortsetzung des Beatmungs-Schlauches 5 gelangt und somit von dem Luftverteiler 7 und dem Tubus 11 die Lunge 23 des Patienten 21 erreicht.

Diese Luftdruckschwankungen führen in der Lunge 23 des Patienten 21 zu einer Schleimlösung, denn der positive und negative Luftdruck löst den möglicherweise in der Lunge 23 vorhandenen Schleim, so dass freibewegliche Schleimpartikel entstehen. Der von dem Beatmungsgerät erzeigte Unterdruck dient zum Absaugen der verbrauchten Atemluft 13 und führt dazu, dass diese Schleimpartikel mit dessen Hilfe aus der Lunge 23 herausgeführt bzw. abtransportiert sind.

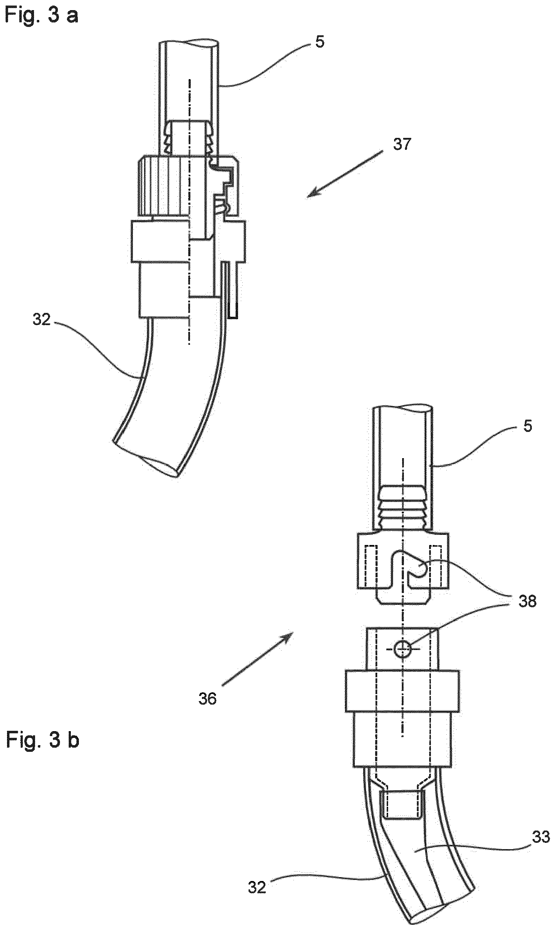

Aus den Figuren 3a und 3b ist ersichtlich, wie das Rohr 32 an dem Beatmungs-Schlauch 5 der Beatmungs-Vorrichtung 1 befestigt werden kann. Zu diesem Zweck sind an den freien Enden des Rohrs 32 jeweils ein Befestigungsadapter 36 und 37 vorgesehen, die fest und luftdicht mit dem Rohr 32 verbunden sind. An den beiden Befestigungsadaptern 36 und 37 ist ein Druckknopf oder ein Stift vorgesehen, der in eine im wesentlichen L-förmigen gestaltete Nute eingesetzt werden kann und in diese nach Art eines Bajonettverschlusses 38 oder einer Hinterschneidung einrasten oder ein geknipst ist.

Die Befestigung des Rohres 32 an dem Beatmungs-Schlauch 5 kann auch mithilfe eines Außen- und Innengewindes erfolgen. Funktionsentscheidend ist ausschließlich, dass das Atemwegstherapiegerät 31 luftdicht mit dem Beatmungs-Schlauch 5 verbunden werden kann und dass das Atemwegstherapiegerät 31 ohne zeitliche Verzögerung und unkompliziert von dem Beatmungsschlauch 5 entfernt bzw. mit diesem verbunden werden kann.

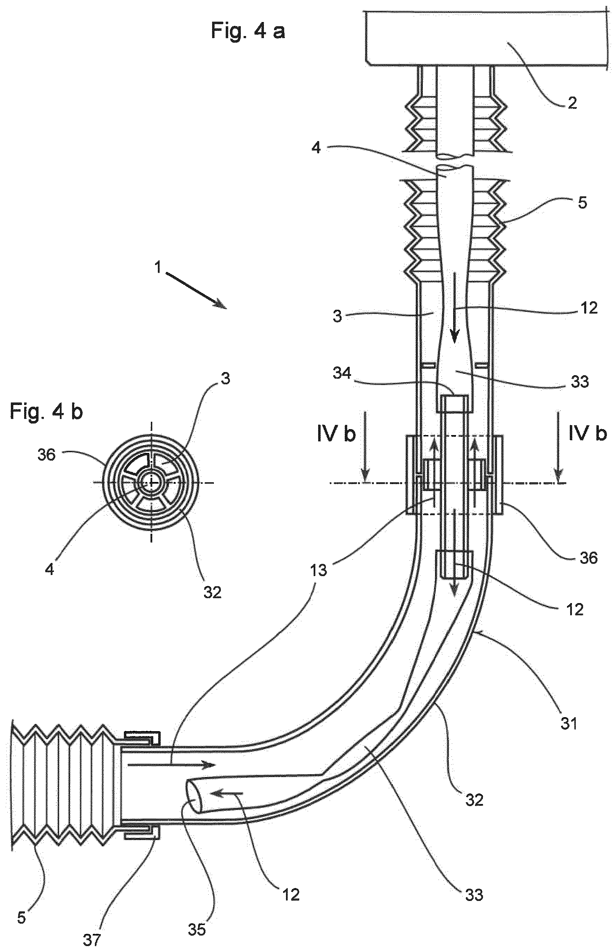

Gemäß den Figuren 4a und 4b liegt der Beatmungsschlauch 5 im Inneren des Ausatmungsschlauches 6, derart, dass zwischen diesen ein Zwischenraum entsteht, durch den die verbrauchte Atemluft 13 durch das Beatmungsgerät 2 gesaugt ist.

Das in den Beatmungsschlauch 5 eingesetzte Atemwegstherapiegerät 31 wird demnach durch den von dem Beatmungsgerät 2 ausgepressten Luftstrom 12 mit diesem beaufschlagt und der Schlauch 33 schwingt innerhalb des Rohres 32. Beim Aussaugen der verbrauchten Atemluft 13 verschließt dieser vollständig, so dass diese Atemluft 13seitlich neben dem Schlauch 33 entlang strömt.

Das Ventil 19 kanalisiert demnach die Sauerstoffzufuhr von dem Beatmungsgerät 2 unmittelbar zu dem Patienten über die Bypass-Leitung 18 oder holt den Sauerstoffstrom durch das Atemwegstherapiegerät 31. Das Ventil 19 kann manuell von außen in diese beiden Stellungen überführt werden und bildet somit eine Art Abzweigung für den Sauerstoffstrom.

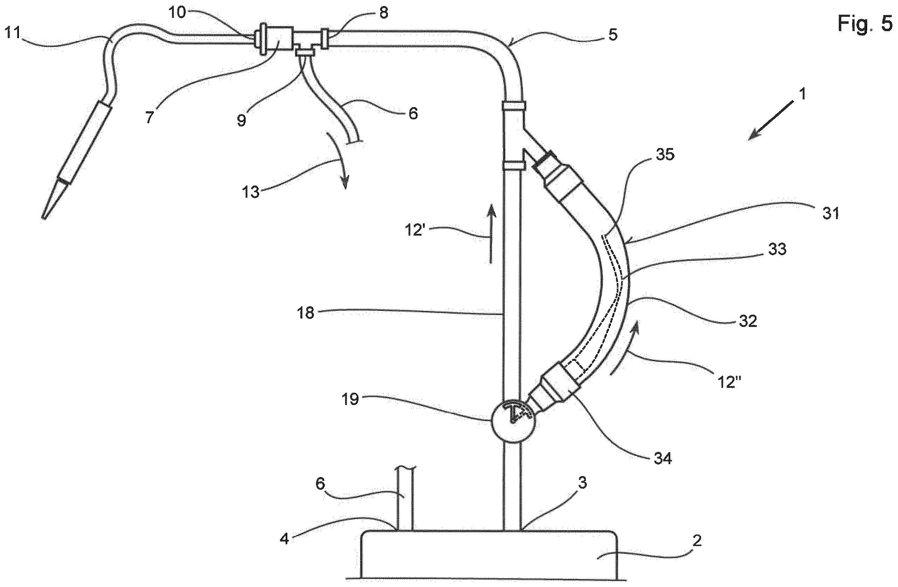

Aus Figur 5 ist das Atemwegstherapiegerät 31 über eine Bypass-Leitung 18 von dem Beatmungskreislauf zu entkuppeln, wenn ein in dieses eingesetztes Ventil 19 verschlossen ist. Sobald das Ventil 19 den Luft-Zugang in das Atemwegstherapiegerät 31 verschließt, strömt der Sauerstoff aus dem Beatmungsgerät 2 unmittelbar durch eine Bypass-Leitung 18 und das Atemwegstherapiegerät 31 übt keine Funktion aus. Sollte der Patient auch mit dem Effekt des Atemwegstherapiegerätes 31 versorgt werden, ist das Ventil 19 derart einzustellen, dass der Sauerstoff aus dem Beatmungsgerät 2 in das Rohr 32 des Atemwegstherapiegerätes 31 strömt und den Schlauch 33 in Schwingung versetzt.

Die erfindungsgemäße Vorrichtung 1 erzeugt soweit bei allen beschriebenen Ausführungsvariante eine intermittierende Luftdruck-Schwankung, die in die Bronchien bzw. Lungenbereiche des Lebewesens 21 weitergeleitet sind und dort die Schleimlösung bewirken.

## Patentansprüche

1. Beatmungs-Vorrichtung (1) für Lebewesen (21), die keine selbstständige Atmung besitzen und die medizinische Hilfe in Form einer künstlichen oder maschinellen Beatmung benötigen, bestehend aus:
- einem Beatmungsgerät (2) mit einem Luftausgangs- (3) und einem Lufteingangs-Kanal (4),
- zwei Schläuchen (5, 6), die mit einer der beiden Kanälen (3, 4) des Beatmungsgerätes (2) verbunden sind,
- einem Luftverteiler (7), an dem drei Öffnungen (8, 9, 10) vorgesehen sind, die mit einer der beiden Schläuche (5, 6) des Beatmungsgerätes (2) und mit einem in den Mund- oder Nasenraums (22) eines Lebewesens (21) einsetzbaren Tubus (11) gekoppelt sind,
**dadurch gekennzeichnet,**
**dass** in dem Einatmungsschlauch (5) ein Atemwegstherapiegerät (31) eingesetzt ist, durch das Schleimablagerungen gelöst und abtransportiert werden,
**dass** durch das Beatmungsgerät (2) ein Luftstrom (12) generiert ist, durch den mit dem Atemwegstherapiegerät (31) eine intermittierende Luftdruck-Schwankung entsteht, und dass die derart erzeugten Luftdruck-Schwankungen in dem Lungenbereich (23) der Lebewesens (21) weitergeleitet oder übertragen sind,
**dass** das Atemwegstherapiegerät (31) aus einem gekrümmten Rohr (32) besteht, das luftdicht in den Beatmungsschlauch (5) integriert oder mit diesem verbunden ist,
**dass** an der Atemluft-Eingangsseite (34) des Atemwegstherapiegerätes (31) ein biegeelastischer Schlauch (33) befestigt ist und dass das gegenüberliegende Ende (35) des Schlauches (33) in dem Rohr (32)und durch den eingepressten Luftstrom (12) oszillierend schwingt.
**dass** das Atemwegstherapiegerät (31) aus einem gekrümmten Rohr (32) besteht, das luftdicht in den Beatmungsschlauch (3) integriert oder mit diesem verbunden ist,
und **dass** innerhalb des Rohrs (32) ein Ventil in Form einer Drosselklappe oder einer Kugel eingesetzt ist, die durch den eingepressten Luftstrom (12) in eine oszillierende Schwingung versetzt ist.

2. Vorrichtung nach Anspruch 1,
**dadurch gekennzeichnet,**
**dass** an dem Beatmungsschlauch (5) zwei Befestigungsadapter (36, 37) vorgesehen sind, an denen das Atemwegstherapiegerät (31) nach Art eines Bajonett-Verschlusses (38), einem Gewinde oder mittels Druckknöpfen arretiert ist.

3. Vorrichtung nach Anspruch 2,
**dadurch gekennzeichnet,**
**dass** die beiden Befestigungsadapter (36, 37) miteinander luftdicht verrastbar oder an einander befestigbar sind.

4. Vorrichtung nach einem der vorgenannten Ansprüche,
**dadurch gekennzeichnet,**
**dass** der Beatmungsschlauch (5) von dem Ausatmungsschlauch (6) umschlossen ist und dieser mittels eines Ventils den von dem Beatmungsgerät (2) ausgepressten Luftstrom (12) in den Beatmungsschlauch (6) und anschließend in das Atemwegstherapiegerät (31) gelangt.

5. Vorrichtung nach Anspruch 4,
**dadurch gekennzeichnet,**
**dass** der Schlauch (33) des Atemwegstherapiegerätes (31) durch den Ausatmungsdruck des Beatmungsgerätes (2) nach Art eines Ventils verschlossen ist und dass die verbrauchte Atemluft (13) seitlich neben diesem Schlauch (33) in Richtung des Beatmungsgerätes (2) geleitet ist.

6. Vorrichtung nach einem der vorgenannten Ansprüche,
**dadurch gekennzeichnet,**
**dass** parallel zu dem Atemwegstherapiegerät (31) eine Bypass-Leitung (18) vorgesehen ist, in die ein Ventil (19) eingesetzt ist, und dass durch das Ventil (19) die Bypass-Leitung (18) geöffnet oder verschlossen ist und das Atemwegstherapiegerät (31) an dem Beatmungsgerät (2) angeschlossen oder von diesem getrennt ist.

7. Vorrichtung nach Anspruch 6,
**dadurch gekennzeichnet,**
**dass** das Ventil (19) eine Art Abzweigung für den Sauerstoffstrom aus dem Beatmungsgerät (22) zu dem Lebewesen (21) bildet und dass das Ventil (19) von außen manuell verstellbar ist.

## Claims

1. Ventilation device (1) for living beings (21), who cannot breathe self-dependently and need medical assistance in the form of artificial or machine ventilation, consisting of:
- a ventilator (2) having an air outlet channel (3) and an air inlet channel (4),
- two hoses (5, 6) connected with one of the two channels (3, 4) of the ventilator (2),
- an air distributor (7) at which three openings (8, 9, 10) are provided that are coupled with one of the two hoses (5, 6) of the ventilator (2) and with a tubus (11), which can be inserted into the oral or nasal area (22) of a living being (21),
**characterised in that**
an airway therapy device (31) is inserted in the inhalation hose (5) by means of which mucus is loosened and removed,
the ventilator (2) generates an airflow (12) by means of which an intermitting air pressure fluctuation is created through the airway therapy device (31) and **in that** the air pressure fluctuation generated is passed on or transmitted into the lung area (23) of the living being (21),
the airway therapy device (31) consists of a curved tube (32) that is integrated into the ventilation hose (5) or connected therewith in an airtight manner,
a flexurally elastic hose (33) is mounted to the breathing-air entrance side (34) of the airway therapy device (31) and **in that** the opposite end (35) of the hose (33) oscillates in the tube (32) and through the injected airflow (12),
the airway therapy device (31) consists of a curved tube (32) that is integrated into the ventilation hose (3) or connected therewith in an airtight manner,
and that within the tube (32), a valve in the form of a throttle valve or a ball is inserted that is caused to oscillate by the injected airflow (12).

2. The device according to claim 1,
**characterised in that**
at the ventilation hose (5) two fixation adapters (36, 37) are provided, to which the airway therapy device (31) is fixedly mounted in the form of a bayonet connection (38), a threaded connection or by means of push buttons.

3. The device according to claim 2,
**characterised in that**
the two fixation adapters (36, 37) are engageable with or connectable to one another in an airtight manner.

4. The device according to one of the preceding claims, **characterised in that**
the ventilation hose (5) is encompassed by the exhalation hose (6) and that, by means of a valve, it transports the airflow (12) pressed out from the ventilator (2) into the ventilation hose (6) and subsequently into the airway therapy device (31).

5. The device according to claim 4,
**characterised in that**
the hose (33) of the airway therapy device (31) is closed in a valve-like manner by the exhalation pressure of the ventilator (2) and that the used breathing air (13) is conducted laterally alongside this hose (33) in the direction of the ventilator (2).

6. The device according to one of the preceding claims, **characterised in that**
in parallel to the airway therapy device (31) a bypass conduit (18) is provided, into which a valve (19) is inserted and **in that** through the valve (19) the bypass conduit (18) is opened or closed and the airway therapy device (31) is connected to the ventilator (2) or disconnected therefrom.

7. The device according to claim 6,
**characterised in that**
the valve (19) forms some kind of a branch line for the oxygen current from the ventilator (22) to the living being (21) and that the valve (19) is manually adjustable from outside.

## Revendications

1. Appareil respiratoire (1) pour êtres vivants (21) sans respiration indépendante et qui ont besoin d'une assistance médicale sous la forme d'une respiration artificielle ou mécanique, comprenant :
- un appareil respiratoire (2) avec un canal d'évacuation d'air (3) et un canal d'entrée d'air (4),
- deux tuyaux flexibles (5, 6) raccordés à un des deux canaux (3, 4) de l'appareil respiratoire (2),
- un distributeur d'air (7) sur lequel il est prévu trois ouvertures (8, 9, 10) raccordés à un des deux tuyaux flexibles (5, 6) de l'appareil respiratoire (2) et à un tube (11) se laissant insérer dans la cavité buccale ou nasale (22) d'un être vivant (21),
**caractérisé en ce que**
dans le tuyau flexible de respiration (5), il est disposé un appareil de thérapie pour voies respiratoires (31) permettant de détacher et d'évacuer des dépôts muqueux,
le respirateur (2) produit un courant d'air (12) qui, en rapport avec l'appareil de thérapie pour voies respiratoires (31), produit une fluctuation intermittente de la pression d'air et que les fluctuations de la pression d'air ainsi générées sont dirigées ou transférées aux poumons (23) de l'être vivant (21), l'appareil de thérapie pour voies respiratoires (31) consiste d'un tuyau coudé (32) intégré de manière étanche à l'air dans le tuyau flexible de respiration (5) ou raccordé à celui-ci,
sur le côté d'entrée de (34) de l'air respirable de l'appareil de thérapie pour voies respiratoires (31), il est fixé un tuyau flexible élastique (33) et que grâce au courant d'air refoulé (12), l'extrémité opposée (35) du tuyau flexible (33) oscille dans le tuyau (32), et que
dans le tuyau (32), il est inséré une soupape sous la forme d'un clapet d'étranglement ou d'une bille mise en oscillation par le courant d'air refoulé (12).

2. Dispositif d'après la revendication 1,
**caractérisé en ce que**
sur le tuyau flexible de respiration (5), il est prévu deux adaptateurs de fixation (36, 37) sur lesquelles l'appareil de thérapie pour voies respiratoires (31) est fixé à l'aide d'une fermeture à baïonnette (38), d'un filetage ou moyennant des boutons poussoirs.

3. Dispositif d'après la revendication 2,
**caractérisé en ce que**
les deux adaptateurs de fixation (36, 37) se laissent cranter ou fixer de manière étanche à l'air l'un sur l'autre.

4. Dispositif d'après une des revendications précédentes,
**caractérisé en ce que**
le tuyau de respiration (5) est entouré par le tuyau d'évacuation (6) et que, moyennant une soupape, celui-ci presse le courant d'air (12) chassé par l'appareil respiratoire (2), dans le tuyau de respiration (6) et ensuite dans l'appareil de thérapie pour voies respiratoires (31).

5. Dispositif d'après la revendication 4,
**caractérisé en ce que**
la pression d'exhalation de l'appareil respiratoire (2) assure la fermeture du tuyau flexible (33) de l'appareil de thérapie pour voies respiratoires (31) à la manière d'une soupape et que l'air vicié (13) est dirigé sur le côté de ce tuyau flexible (33) en direction de l'appareil respiratoire (2).

6. Dispositif d'après une des revendications précédentes,
**caractérisé en ce que**
parallèlement à l'appareil de thérapie pour voies respiratoires (31), il est prévu une conduite by-pass (18) dans laquelle il est monté une soupape (19) et que la soupape (19) assure l'ouverture ou la fermeture de la conduite bypass (18), l'appareil de thérapie pour voies respiratoires (31) étant raccordé ou séparé de l'appareil respiratoire (2).

7. Dispositif d'après la revendication 6,
**caractérisé en ce que**
la soupape (19) constitue une bifurcation dirigeant le flux d'oxygène en provenance de l'appareil respiratoire (2) vers l'être vivant (21) et que la soupape (19) se laisse régler manuellement de l'extérieur.
